# FASCICULE DE BREVET EUROPEEN

(11) **EP 2 037 896 B1**
(45) Date de publication et mention de la délivrance du brevet: **04.05.2016**
(21) Numéro de dépôt: 07803765.2
(22) Date de dépôt: 21.06.2007
(51) Int. Cl.: A61K 9/46, A61K 8/02, A61K 9/16, A61K 8/97, A61K 8/81, A61K 8/73, A61Q 19/00

(54) **GRANULES OU GRANULÉS EFFERVESCENTS**
BRAUSEGRANULAT ODER BRAUSEGRANULATMATERIAL
EFFERVESCENT GRANULES OR GRANULAR MATERIAL

(30) Priorité: 21.06.2006 FR 0605549
(43) Date de publication de la demande: 25.03.2009
(73) Titulaire: VIRBAC S.A., F-06510 Carros (FR)
(72) Inventeur: DERRIEU, Guy, F-06800 Cagnes sur Mer (FR)
(74) Mandataire: Macquet, Christophe
(86) Numéro de dépôt international: PCT/FR2007/001027
(87) Numéro de publication internationale: WO 2007/147976

(56) Documents cités:
- GB-A- 2 217 598
- US-A- 2 984 543
- US-A1- 2004 057 995

## Description

La présente invention concerne des granules ou des granulés effervescents stables comprenant au moins un composé générateur d'effervescence. L'invention concerne en outre des utilisations de tels granules ou granulés ainsi que des aliments supplémentés incorporant ces granules ou granulés. Les granules ou granulés selon l'invention sont destinés à être dilués dans de l'eau pour ensuite, par exemple, être administrés par voie orale ou à usage topique.

L'obtention de compositions effervescentes formulées, c'est à dire de compositions telles que des granules ou granulés qui, à la différence d'un simple mélange de poudres, ont subi un changement de forme, et qui sont stables physiquement et chimiquement dans le temps, est une opération difficile à réaliser. En effet, de telles compositions perdent leur effervescence au cours de leur conservation. Ceci est dû, notamment, à l'initiation de la réaction qui conduit à l'effervescence en présence d'eau pendant les étapes de formulation des compositions et de leur mise en forme. C'est par exemple le cas des compositions effervescentes formulées divulguées dans les documents US 5,853,759 et EP 1 140 035.

Afin de compenser et de limiter la perte d'effervescence, un moyen classiquement retenu est d'augmenter la quantité de composés effervescents. Ainsi, en fin de conservation, une effervescence est toujours présente, même si elle est nettement moins importante qu'à l'origine. Une telle solution n'est cependant pas envisageable quand la quantité d'agent effervescent à introduire est faible par rapport aux autres constituants de la composition.

Un autre moyen retenu pour limiter la perte d'effervescence consiste à éviter de déclencher la réaction d'effervescence en préparant les granules ou granulés à sec, c'est-à-dire sans la présence d'eau ou d'humidité. Cet autre moyen est mis en oeuvre, par exemple, dans les documents WO 9116893 et WO 9115197. En pratique, il est effectivement souhaitable de préparer les granules et granulés à sec lorsque les constituants de ceux-ci sont sensibles à l'humidité. Cela impose par contre de n'utiliser que des liants solubles dans des solvants autres que l'eau, comme les alcools par exemple, pour effectuer des granulation humides. Toutefois, bien souvent, les granules ou granulés préparés à sec ne présentent pas une cohésion suffisante pour éviter un retour à la forme pulvérulente de départ. Or, le maintien de l'intégrité des granules ou granulés obtenus par granulation ou compactage permet une meilleure dissolution de la composition dans l'eau. Cette intégrité est en outre requise par les consommateurs de granules ou granulés.

La demande US 2004 57995 décrit un procédé de fabrication de comprimés orodispersibles préférentiellement effervescents comprenant de la maltodextrine. La demande GB 2217598 décrit, quant à elle, un produit fini qui est un comprimé effervescent capable de se désintégrer rapidement pour produire une suspension aqueuse, adaptée à une administration orale et comprenant du Diclofénac micronisé enrobé d'un revêtement perméable à l'eau masquant l'amertume de l'actif. La demande US 2984543 aborde le problème de la stabilisation d'agents effervescents par ajout de gommes, notamment dans les domaines des médicaments et des boissons gazeuses.

Considérant ce qui précède, un problème que se propose de résoudre l'invention est de réaliser des granules ou des granulés effervescents, qui présentent un bon maintien de leur intégrité ainsi qu'une stabilité améliorée de leur effervescence dans le temps.

La solution proposée par l'invention a pour premier objet des granules ou granulés effervescents comprenant un composé générateur d'effervescence, caractérisés en ce qu'ils comprennent en outre au moins une gomme préférentiellement biocompatible et 1,0 à 50 mg d'eau par g de granules ou granulés.

Elle a pour second objet l'utilisation de tels granules ou granulés dans le domaine pharmaceutique, vétérinaire, cosmétique, agricole ou horticole, de l'hygiène, ou de l'entretien, ainsi que leur utilisation pour la préparation d'aliments supplémentés liquides pour animaux dans lesquels ils sont incorporés.

Enfin, elle a pour troisième objet un aliment supplémenté pour animaux incorporant de tels granules ou granulés.

Ainsi que cela a été expliqué plus haut, l'homme du métier sait que la présence d'eau a pour effet d'initialiser la réaction d'effervescence. Il en déduit que les gommes selon l'invention, en particulier les gommes naturelles ou non, qui présentent un pouvoir collant en présence d'eau mais qui ne sont pas ou peu solubles dans des solvants biocompatibles, ne devraient pas pouvoir être utilisées pour améliorer la stabilité et la cohésion de granules ou granulés effervescents. En réalisant des granules ou granulés comportant à la fois de l'eau, au moins une gomme et un composé générateur d'effervescence, l'invention va donc à l'encontre de ce préjugé de l'homme du métier.

L'invention sera mieux comprise à la lecture de la description non limitative qui va suivre.

Les granules ou granulés selon l'invention comprennent un ou plusieurs composés générateurs d'effervescence. Ces composés peuvent être choisis parmi : - les couples associant une source acide choisie parmi l'acide citrique, l'acide tartrique, l'acide malique, l'acide fumarique, l'acide adipique, l'acide succinique, les anhydrides desdits acides, un sel d'acide choisi parmi le phosphate monosodique, le pyrophosphate disodique ou le sulfite sodique, les mélanges de ces acides, anhydrides, et les sels acides ; et une source carbonate choisie parmi le bicarbonate de sodium, le carbonate de sodium, le bicarbonate de potassium, le carbonate de potassium, le sesquicarbonate de sodium, les dérivés d'aminoacides tels que ceux divulgués dans le brevet US 3,392,195 comme le bicarbonate de glycine sodique, le bicarbonate de diglycine sodique, le bicarbonate de glycine sodique, le carbonate de diglycine sodique, le bicarbonate d'arginine sodique, et les mélanges de ces derniers ou pour certains seul ; ou - le percarbonate de sodium, le perborate de sodium ou le carbonate de sodium.

Le ou les composés générateurs d'effervescence sont préférentiellement présents à la concentration au minimum de 50 mg par g de granules ou granulés. Encore plus préférentiellement, la concentration de tels composés est au minimum de 100 mg par g de granules ou granulés.

Dans les compositions selon l'invention, la ou les gommes sont des gommes naturelle (s) ou non, peu ou pas solubles dans les solvants biocompatibles autres que l'eau, c'est-à-dire, principalement, dans les alcools volatiles comme l'alcool éthylique ou l'alcool isopropylique. Elles présentent en outre un pouvoir collant en présence d'eau, de manière à réaliser la cohésion des granules ou granulés.

Ces gommes peuvent être choisies parmi les gommes végétales comme la gomme d'acacia produite par Acacia *Senega*™ et Acacia *Seyal*™*,* les carraghénanes, les alginates, les pectines, la gomme karaya, la gomme xanthane, ou le chitosan ou ses dérivés, l'acide hyaluronique, ou autres comme les polymères pullulan ou agar, ou le dextran, ou les celluloses et leurs dérivés tels que la methylcellulose ou l'hydroxypropylcellulose, les polymères de l'acide acrylique réticulés avec un éther allylique, tels que ceux connus sous le nom générique de carbomers, essentiellement constitués d'un carbomer ou d'un mélange de carbomers tels que les polymères de l'acide acrylique réticulés avec des polyalkenyl éthers fabriqués par B.F. Goodrich™ sous la dénomination Carbopol™.

La ou les gommes sont préférentiellement présentes, dans les granules ou granulés effervescents, à la concentration au minimum de 1 mg par g de granules ou granulés. Préférentiellement, cette concentration est au minimum de 10 mg par g de granules ou granulés. Encore plus préférentiellement, elle est au minimum de 40 mg par g de granules ou granulés.

La quantité d'eau présente dans les granules ou granulés selon l'invention est comprise entre 1,0 et 50 mg par g de granules ou granulés, préférentiellement, entre 1,0 et 30 mg par g de granules ou granulés et, plus préférentiellement, entre 10 et 30 mg par g de granules ou granulés.

Les solvants biocompatibles autres que l'eau peuvent être choisis parmi les solvants utilisés pour la granulation de composés dans les domaines pharmaceutique, vétérinaire, cosmétique, de l'élevage, agricole ou horticole, de l'hygiène, ou alors, de l'entretien. Ils sont préférentiellement volatiles. Il s'agit par exemple de l'alcool éthylique, de l'alcool propylique ou de l'alcool isopropylique. Le solvant préférentiellement retenu est l'alcool isopropylique.

Les granules ou granulés selon l'invention peuvent par ailleurs contenir d'autres composés, dits composés secondaires, acceptables seuls ou associés dans le domaine envisagé. A titre d'exemple dans le domaine pharmaceutique ou vétérinaire, il peut s'agir de composés de charge comme les sucres tels que le lactose, le saccharose ou les maltodextrines, comme les polyols tels que le sorbitol ou le mannitol, comme l'amidon, la cellulose microcristalline, un phosphate, des agents conservateurs comme l'acide benzoïque ou ses sels, de préférence le sel de sodium, ou l'acide para hydroxybenzoïque, ou les esters de l'acide parahydroxybenzoïque de préférence les esters méthylique, éthylique, propylique ou butylique, ou leurs sels, de préférence les sels de sodium, utilisés seuls ou en association, l'acide sorbique ou ses sels, de préférence le sel de potassium, ou le chlorbutanol, des agents de mouillage tels que les anioniques comme le docusate de sodium ou le sulfate de lauryle sodique, des agents tensioactifs, tels que les non ioniques comme les polysorbates ou les polyoxamers, des agents viscosants tels que les gommes comme la gomme adragante ou les gommes d'acacia ou les carraghénanes ou l'acide alginique et ses sels, la gomme karaya, la gomme xanthane, ou le chitosan ou ses dérivés, des substances constituant un tampon comme les phosphates ou les citrates tels que les phosphates sodiques ou potassiques ou l'acide citrique ou ses sels, des agents osmotiques comme le chlorure de sodium ou le mannitol, des lubrifiants comme le dioctylsulfosuccinate de sodium, les polyéthylène glycols et leurs dérivés, le laurylsulfate de sodium, le benzoate de sodium.

Les granules ou granulés selon l'invention peuvent également contenir différents additifs tels que des arômes, des agents parfumants, des édulcorants appétibles, des colorants.

Les granules ou granulés effervescents selon l'invention trouvent des applications dans de nombreux domaines tels que les domaines alimentaire, pharmaceutique, vétérinaire, cosmétique, de l'élevage, agricole ou horticole, de l'hygiène, de l'entretien.

Dans le domaine cosmétique, pharmaceutique et/ou vétérinaire, les granules ou granulés effervescents selon l'invention contiennent un ou plusieurs principes actifs exerçant une activité pharmacologique. Par exemple, les granules ou granulés selon l'invention sont susceptibles de comprendre une ou plusieurs vitamines, minéraux et/ou oligo-éléments.

Par ailleurs, les granules ou granulés selon l'invention sont susceptibles de comprendre un ou plusieurs antibiotiques hydrosolubles à action générale ou locale. On peut citer, à titre d'exemple non limitatif, comme antibiotique : le chlorhydrate de tétracycline. Les granules ou granulés selon l'invention peuvent aussi comprendre un ou plusieurs agents anthelminthiques ou anti-protozoaires hydrosolubles. On peut citer à titre d'exemple non limitatif l'ivermectine, la milbemycine ou le fipronil.

Dans le domaine pharmaceutique ou cosmétique notamment, les granules ou granulés effervescents selon l'invention peuvent être destinés à une administration par voie orale ou à un usage topique après dissolution dans de l'eau. Ils peuvent aussi subir une ou plusieurs étapes de formulation supplémentaires en vue d'obtenir une forme galénique plus adaptée à l'administration. En particulier, à titre d'exemple non limitatif, les granules ou granulés effervescents selon l'invention peuvent subir un compactage pour une mise en forme de comprimés effervescents en évitant une trop forte force de compression, non nécessaire par la présence du liant afin de ne pas nuire à la vitesse de dissolution.

L'homme du métier veillera à ce que les constituants individuels des granules ou granulés selon l'invention soient compatibles entre eux.

Selon un mode de réalisation, les granules ou granulés selon l'invention sont préparés par granulation au sein d'un granulateur. Pour ce faire, l'ensemble des composants pulvérulents, y compris la ou les gommes, et à l'exception, éventuellement, d'un composé générateur d'effervescence (un membre du couple acide/carbonate lorsqu'il s'agit d'un couple), sont tout d'abord mélangés, soit au préalable, soit dans le granulateur quand il le permet, puis on effectue une pulvérisation d'un solvant organique dans lequel a été introduite, éventuellement, la quantité d'eau retenue, cette quantité d'eau étant susceptible de provenir par ailleurs des composants eux-mêmes (eau résiduelle). Le solvant organique est notamment un alcool, de préférence l'isopropanol. Après élimination de ce solvant par évaporation, des granules ou granulés sont ainsi obtenus.

Selon un autre mode de réalisation, les granules ou granulés selon l'invention sont préparés au moyen d'un granulateur à lit d'air fluidisé dans lequel la composition est mise en suspension dans un courant d'air. Un solvant organique non aqueux dans lequel à été introduite la quantité d'eau retenue tel que décrit précédemment est alors pulvérisé au moyen d'une buse. Après élimination du solvant par évaporation des granules ou granulés ainsi obtenus, les agglomérés résultants sont calibrés.

Selon encore un autre mode de réalisation, les granules ou granulés sont préparés par compactage au moyen d'un compacteur dans lequel la composition, après avoir été mélangée et sur laquelle la quantité d'eau a été éventuellement très finement pulvérisée, est immédiatement introduite dans le compacteur. Les compactés résultants sont broyés puis calibrés.

L'invention inclut également, comme technique de granulation ou de compactage, toute autre technique connue de l'homme du métier permettant d'obtenir le même résultat.

L'invention concerne un procédé de fabrication de granulés effervescents comprenant au moins une gomme, naturelle ou non, peu ou pas solubles dans les solvants biocompatibles autres que l'eau, au moins un composé générateur d'effervescence et de 1,0 à 50 mg d'eau par g de la composition comprenant une étape de pulvérisation d'un solvant organique non aqueux dans lequel a été introduite la quantité d'eau retenue.

L'invention concerne aussi un tel procédé de fabrication de granules ou granulés effervescents mis en oeuvre au moyen d'un granulateur à lit d'air fluidisé, le solvant organique non aqueux dans lequel a été introduite la quantité d'eau retenue étant pulvérisé sur la composition mise en suspension dans un courant d'air au moyen d'une buse.

L'invention concerne par ailleurs un procédé de fabrication de granules ou granulés effervescents comprenant au moins une gomme, naturelle ou non, peu ou pas solubles dans les solvants biocompatibles autres que l'eau, au moins un composé générateur d'effervescence et de 1,0 à 50 mg d'eau par g de granules ou granulés par compaction au moyen d'un compacteur dans lequel la quantité d'eau est finement pulvérisée sur le mélange des autres constituants avant compaction, les compactés résultants sont ensuite broyés et calibrés.

Les produits obtenus après granulation ou compactage et broyage, ont un diamètre supérieur à 50 *µ*m et inférieur à 6 mm, préférentiellement compris entre 150 *µ*m et 1 mm pour les granules et entre 2 mm et 6 mm pour les granulés.

Dans le domaine de l'alimentation animale et de la thérapeutique vétérinaire, la présente Invention a aussi pour objet l'utilisation des granules ou granulés effervescents selon l'invention pour la préparation d'aliments supplémentés liquides (aqueux) ou de l'eau de boisson supplémentée pour animaux.

Dans le milieu aquatique tel que les aquariums, mares ou bassins, on introduit les granules ou les granulés tels quels ce qui permet de traiter les organismes aquatiques (poissons, crustacés, plantes aquatiques, mollusques algues ...) par balnéation. De même on introduit les granules ou les granulés tels quels dans de l'eau pour les produits d'entretien, les cosmétiques comme les sels de bains, les produits agricoles ou horticoles.

Des études ont été réalisées afin de mettre en évidence les avantages des granules ou granulés selon l'invention, ainsi que leurs procédés de préparation. Les résultats de ces études sont reportés dans les exemples ci-après. Ceux-ci ne sont donnés qu'à titre d'illustration et ne doivent pas être considérés comme limitant l'invention.

### EXEMPLE 1 : Préparation de granulés effervescents conformément à l'invention (granulés N°1) et comparatif avec des granulés préparés selon l'art antérieur (granulés N°2)

Les granulés N°1 et N°2 sont préparés à partir des composants listés dans le tableau suivant :

| COMPOSANTS | GRANULES | |
|---|---|---|
| | N°1 | N°2 |
| Chlorure de sodium | 4,70 g | 4,70 g |
| Chlorure de potassium | 3,00 g | 3,00 g |
| Bicarbonate de sodium | 13,40 g | 13,40 g |
| Acide citrique | 7,80 g | 7,80 g |
| Lactose raffiné | 47,73 g | 47,73 g |
| Glycine | 4,58 g | 4,58 g |
| Ethyl vanilline | 0,03 g | 0,03 g |
| Poudre lactosérum spray | 10,00 g | 10,00 g |
| Gomme d'acacia | 4,00 g | 4,00 g |
| Farine de riz | 1,00 g | 1,00 g |
| Farine de caroube | 1,00 g | 1,00 g |
| Sélenomethionine (2000 ppm) | 0,50 g | 0,50 g |
| Vit E | 1,76 g | 1,76 g |
| Bactéries lactiques | 0,50 g | 0,50 g |

Pour les granulés N°1, on mélange mécaniquement les différents composants à l'exception du bicarbonate de sodium. L'eau est vaporisée très finement sur le mélange sous agitation. Quant le mélange est apparemment sec (pas de trace d'eau apparente), on ajoute, dans une étape ultérieure au mélange de la gomme et de l'eau, le bicarbonate de sodium. La granulation est effectuée par vaporisation de l'alcool isopropylique. Une fois que la granulation est terminée, le solvant est chassé sous vide avec un léger chauffage. Il apparaît que le teneur en eau des granulés, déterminée par perte à la dessiccation, est de 15 mg/g de granulés.

Pour les granulés N°2, on mélange mécaniquement l'ensemble des composants. Une fois que le mélange est réalisé, la granulation est effectuée par vaporisation de l'alcool isopropylique. Lorsque la granulation est terminée, le solvant est chassé sous vide avec un léger chauffage. On observe immédiatement que la granulation a été incomplète et que la composition se présente sous forme pulvérulente.

### EXEMPLE 2 Préparation de granules effervescents par compactage et broyage selon l'invention (granulés N°3) et comparatif avec des granules préparés selon l'art antérieur (granulés N°4)

Les granules N°3 et N°4 sont préparés à partir des composants listés dans le tableau suivant :

| COMPOSANTS | GRANULES | |
|---|---|---|
| | N°3 | N°4 |
| Laponite | 3,2 g | 3,2 g |
| Chlorure de potassium | 0,022 g | 0,022 g |
| Iodure de potassium | 0,0016 g | 0,0016 g |
| Bromure de sodium | 0,0032 g | 0,0032 g |
| Chlorure de magnésium | 0,042 g | 0,042 g |
| Sulfate de magnésium | 0,028 g | 0,028 g |
| Bicarbonate de sodium | 17,816 g | 17,816 g |
| Acide citrique anhydre | 10,184 g | 10,184 g |
| Percarbonate de sodium | 30,0 g | 30,0 g |
| Lactose | 29,7032 g | 29,7032 g |
| Gomme d'acacia | 4,0 g | 4,0 g |
| PEG 6000 | 5,0 g | 5,0 g |

Pour la préparation des granules N°3, on mélange mécaniquement les différents composants à l'exception du bicarbonate de sodium. L'eau est vaporisée très finement sur le mélange sous agitation. Quand le mélange est apparemment sec, c'est-à-dire lorsque qu'il n'y a pas de trace d'eau apparente, le compactage est effectué. Les produits compactés sont ensuite broyés puis calibrés par passage sur une grille de 4 mm. Il apparaît que la teneur en eau des granules, déterminée par perte à la dessiccation, est de 27 mg / g de granules.

Pour les granules N°4, on mélange mécaniquement les différents composants à l'exception du bicarbonate de sodium. Le compactage est effectué. Les produits compactés sont ensuite broyés et calibrés par passage sur une grille de 4 mm. On remarque immédiatement qu'au broyage on obtient presque exclusivement de la poudre.

### EXEMPLE 3 Evaluation de l'effervescence des granulés N°1.

L'évaluation de l'effervescence des granulés N°1 de l'Exemple 1 est effectuée par détermination du temps d'émission de bulles de gaz de 10 granulés de poids équivalent, voisin de 10 mg, mis dans 100 ml d'eau du robinet. A chaque temps de conservation, le résultat est exprimé en pourcentage, représentant le rapport du temps d'effervescence au temps envisagé par rapport au temps initial. On obtient alors le tableau suivant :

| TEMPS DE CONSERVATION | % |
|---|---|
| t initial | 100 |
| t 14 jours | 96 |
| t 1 mois | 94 |
| t 2 mois | 95 |

Il apparaît que les granulés N°1 présentent une bonne stabilité dans le temps. Après 2 mois, 95% de l'effervescence initiale est conservée.

### EXEMPLE 4 Evaluation de l'effervescence des granules N°3.

L'évaluation de l'effervescence des granules N°3 obtenus par compactage suivi d'un broyage et calibrage, a été réalisée par détermination du temps d'émission de bulles de gaz de 5 granules de poids équivalent, voisin de 150 mg, mis dans 100 ml d'eau du robinet. A chaque temps de conservation, le résultat est exprimé en pourcentage, représentant le rapport du temps d'effervescence au temps envisagé par rapport au temps initial. On obtient alors le tableau suivant :

| TEMPS DE CONSERVATION | % |
|---|---|
| t initial | 100 |
| t 14 jours | 98 |
| t 1 mois | 97 |
| t 2 mois | 97 |

De même que pour les granulés N°1, il apparaît que les granules N°3 présentent une bonne stabilité dans le temps. Après 2 mois, 97% de l'effervescence initiale est conservée.

En définitive, la présence d'eau dans les granules ou granulés selon l'invention améliore de manière inattendue leurs propriétés physiques telles que la stabilité physique (cohésion ou maintien de l'intégrité de la forme granulée ou compactée), l'effervescence (maintien de la qualité effervescente) et la stabilité chimique de leurs constituants en particulier celle des principes actifs dans le cas des granules ou granulés à usage cosmétique, pharmaceutique ou vétérinaire, d'hygiène ou d'environnement, de l'élevage, agricole ou horticole.

La plus grande stabilité physique due à la cohésion améliorée des granules ou granulés effervescents conduit à une moindre dégradation : moins de production de poudre par effritement des granulés ou granules (qui peuvent être obtenus par granulation ou compactage suivi ou non d'un broyage et calibrage). Il y a ainsi une meilleure conservation de l'intégrité des compositions effervescentes. En outre, les granules et granulés selon l'invention ne perdent pas leur effervescence. Il est ainsi possible de les stocker vendant de plus longues périodes de temps. Enfin, la meilleure stabilité physique et/ou la conservation du caractère effervescent entraînent une meilleure protection et une meilleure stabilité des constituants de ces compositions effervescentes. Ceci est utile pour tous les constituants pour lesquels il s'avère important de garantir la quantité et la qualité apportée par la composition effervescente. En particulier, c'est important pour les principes actifs, notamment vis-à-vis des agressions extérieures dus aux facteurs environnementaux tels que la chaleur ou l'humidité, ou vis-à-vis des opérations éventuelles de transformation (compactage par exemple) des compositions effervescentes sous forme de granulés effervescents ou de granules effervescentes.

## Revendications

1. Granules ou granulés effervescents comprenant
un composé générateur d'effervescence,
au moins une gomme,
**caractérisés en ce que**
la gomme n'est pas ou peu soluble dans des solvants biocompatibles autres que l'eau et présente un pouvoir collant en présence d'eau et est choisie parmi la gomme d'acacia, les carraghénanes, les alginates, les pectines, la gomme karaya, la gomme xanthane, ou le chitosan ou ses dérivés, l'acide hyaluronique, parmi les polymères pullulan ou agar, ou le dextran, ou les celluloses et leurs dérivés, les carbomers, seule ou associée avec d'autres gommes, et **en ce que**
lesdits granules ou granulés effervescents comprennent en outre une quantité d'eau de 1,0 à 50 mg par g de granules ou granulés.

2. Granules ou granulés selon la revendication 1, dans laquelle la gemme est la gomme d'acacia.

3. Granules ou granulés selon l'une des revendications précédentes, dans lesquels la ou les gommes sont présentes à la concentration au minimum de 1 mg par g de granules ou granulés, préférentiellement à la concentration aux minimum de 10 mg par g de granules ou granulés et encore plus préférentiellement à la concentration au minimum de 40 mg par g de granules ou granulés.

4. Granules ou granulés selon l'une des revendications précédentes, dans lesquels le composé générateur d'effervescence représente au moins 50 mg par g de granules ou granulés.

5. Granules ou granulés selon l'une des revendications précédentes comprenant de 10 à 30 mg d'eau par g de granules ou granulés.

6. Granules ou granulés selon l'une des revendications précédentes, comprenant en outre un ou plusieurs composés secondaires.

7. Granules ou granules selon la revendication 6, dans lesquels le composé secondaire est le lactose.

8. Granules ou granulés effervescents selon l'une des revendications précédentes, susceptibles d'être obtenus selon un procédé de fabrication comprenant une étape de pulvérisation d'un solvant organique non aqueux dans lequel a été introduite la quantité d'eau retenue.

9. Granules ou granulés selon la revendication 8, **caractérisés en ce que** le procédé de fabrication est mis en oeuvre au moyen d'un granulateur à lit d'air fluidisé, le solvant organique non aqueux dans lequel a été introduite la quantité d'eau retenue étant pulvérisé sur la composition mise en suspension dans un courant d'air au moyen d'une buse.

10. Granules ou granulés selon l'une des revendications 1 à 7, susceptibles d'être obtenus par compaction au moyen d'un compacteur dans lequel la quantité d'eau est finement pulvérisée sur le mélange des autres constituants avant compaction, les compactés résultants étant ensuite broyés et calibrés.

11. Granules ou granulés selon la revendication 10 pour son application en tant que médicament.

12. Utilisation de granules ou granulés selon l'une quelconque des revendications 1 à 10 dans le domaine cosmétique, ou agricole et horticole, ou de l'hygiène, ou de l'entretien.

13. Utilisation de granules ou granulés selon l'une des revendications 1 à 10, pour la préparation d'aliments supplémentés liquides pour animaux dans lesquels on a incorporé lesdits granules ou granulés.

14. Utilisation de granules ou granulés selon l'une des revendications 1 à 10, pour la préparation d'eau de boisson supplémentée pour animaux dans lesquels on a incorporé lesdits granules ou granulés.

15. Aliment supplémenté pour animaux incorporant des granules ou granulés selon l'une des revendications 1 à 10.

16. Utilisation de granules ou granulés selon l'une des revendications 1 à 10 pour la balnéation d'organismes aquatiques par dissolution desdits granules ou granulés dans le milieu aquatique.

17. Procédé de fabrication de granules ou granulés selon l'une des revendications 1 à 10 par granulation, comportant, dans l'ordre, des étapes de :
mélange de composants pulvérulents desdits granules ou granulés, y compris de la gomme, pour obtenir un mélange ;
pulvérisation, sur ledit mélange, d'un solvant organique dans lequel a été introduite la quantité d'eau ; et
élimination dudit solvant par évaporation, **caractérisé en ce que** le mélange sur lequel est pulvérisé l'eau ne comporte pas de composé générateur d'effervescence ou ne comporte pas de membre du couple de composés générateur d'effervescence lorsqu'il s'agit d'un couple.

## Patentansprüche

1. Brausegranulat oder Brausegranulatmaterial, umfassend eine Verbindung zur Erzeugung von Brause,
mindestens einen Gummi,
**dadurch gekennzeichnet, dass**
der Gummi nicht oder nur wenig löslich in biokompatiblen Lösemitteln außer Wasser ist und in Gegenwart von Wasser eine Haftkraft aufweist und ausgewählt ist aus Gummi arabicum, Carrageenanen, Alginaten, Pektinen, Karayagummi, Xanthangummi oder Chitosan und seinen Derivaten, Hyaluronsäure, aus den Polymeren Pullulan oder Agar, Dextran oder den Zellulosen und ihren Derivaten, den Carbomeren, allein oder assoziiert mit anderen Gummis, und dadurch, dass
das Brausegranulat oder Brausegranulatmaterial außerdem eine Wassermenge von 1,0 bis 50 mg pro g Granulat oder Granulatmaterial umfasst.

2. Granulat oder Granulatmaterial nach Anspruch 1, wobei der Gummi der Gummi arabicum ist.

3. Granulat oder Granulatmaterial nach einem der vorhergehenden Ansprüche, wobei der oder die Gummi in einer Konzentration von mindestens 1 mg pro g Granulat oder Granulatmaterial vorliegen, vorzugsweise in einer Konzentration von mindestens 10 mg pro g Granulat oder Granulatmaterial, oder noch bevorzugter in einer Konzentration von mindestens 40 mg pro g Granulat oder Granulatmaterial.

4. Granulat oder Granulatmaterial nach einem der vorhergehenden Ansprüche, wobei die Verbindung zur Erzeugung von Brause mindestens 50 mg pro g Granulat oder Granulatmaterial darstellt.

5. Granulat oder Granulatmaterial nach einem der vorhergehenden Ansprüche, umfassend 10 bis 30 mg Wasser pro g Granulat oder Granulatmaterial.

6. Granulat oder Granulatmaterial nach einem der vorhergehenden Ansprüche, umfassend außerdem eine oder mehrere sekundäre Verbindungen.

7. Granulat oder Granulatmaterial nach Anspruch 6, wobei die sekundäre Verbindung Laktose ist.

8. Granulat oder Granulatmaterial nach einem der vorhergehenden Ansprüche, das durch ein Herstellungsverfahren erhalten werden kann, umfassend einen Schritt des Zerstäubens eines nicht wässrigen organischen Lösemittels, in das die zurückgehaltene Menge von Wasser eingeführt wurde.

9. Granulat oder Granulatmaterial nach Anspruch 8, **dadurch gekennzeichnet, dass** das Herstellungsverfahren mit Hilfe eines Granulators mit fluidisierten Luftbett durchgeführt wird, wobei das nicht wässrige organische Lösemittel, in das die Menge des zurückgehaltenen Wassers eingeführt wurde, auf der Zusammensetzung, suspendiert in einem Luftstrom mit Hilfe einer Düse, zerstäubt wird.

10. Granulat oder Granulatmaterial nach einem der Ansprüche 1 bis 7, das durch Kompaktierung mit Hilfe einer Kompaktierungsvorrichtung erhalten werden kann, wobei die Menge von Wasser fein auf der Mischung der anderen Bestandteile vor der Kompaktierung zerstäubt wird, wobei die kompaktierten Elemente dann gemahlen und kalibriert werden.

11. Granulat oder Granulatmaterial nach Anspruch 10 für seine Anwendung als Arzneimittel.

12. Verwendung von Granulat oder Granulatmaterial nach einem der Ansprüche 1 bis 10 auf dem Gebiet der Kosmetik oder der Landwirtschaft oder des Gartenbaus oder der Hygiene oder der Unterhaltung.

13. Verwendung von Granulat oder Granulatmaterial nach einem der Ansprüche 1 bis 10 zur Zubereitung von flüssigen ergänzten Nahrungsmitteln für Tiere, in die das Granulat oder Granulatmaterial eingebaut wurde.

14. Verwendung von Granulat oder Granulatmaterial nach einem der Ansprüche 1 bis 10 zur Zubereitung von ergänztem Trinkwasser für Tiere, in die das Granulat oder Granulatmaterial eingebaut wurde.

15. Ergänztes Nahrungsmittel für Tiere, umfassend Granulat oder Granulatmaterial nach einem der Ansprüche 1 bis 10.

16. Verwendung von Granulat oder Granulatmaterial nach einem der Ansprüche 1 bis 10 für die Balneotherapie von Wasserorganismen durch Lösen des Granulats oder Granulatmaterials im wässrigen Milieu.

17. Verfahren zur Herstellung von Granulat oder Granulatmaterial nach einem der Ansprüche 1 bis 10 durch Granulierung, umfassend, in Reihenfolge, die folgenden Schritte:
Mischen von pulverförmigen Bestandteilen des Granulats oder Granulatmaterials, darin eingeschlossen der Gummi, um eine Mischung zu erhalten;
Zerstäuben, auf der Mischung, eines organischen Lösemittels, in das die Menge von Wasser eingeführt wurde; und
Beseitigen des Lösemittels durch Verdampfung, **dadurch gekennzeichnet, dass** die Mischung, auf der das Wasser zerstäubt wird, keine Verbindung zur Erzeugung von Brause umfasst oder kein Glied des Paars von Verbindungen zur Erzeugung von Brause umfasst, wenn es sich um ein Paar handelt.

## Claims

1. Effervescent granules or granular material comprising an effervescence-generating compound,
at least one gum,
**characterised in that**
the gum is insoluble or only slightly soluble in biocompatible solvents other than water and has an adhesive force in the presence of water and is chosen from acacia gum, carrageenans, alginates, pectins, karaya gum, xanthan gum, or chitosan or derivatives thereof, hyaluronic acid, among polymers pullulan or agar, or dextran, or celluloses and derivatives thereof, carbomers, alone or in combination with other gums, and **in that** said effervescent granules or granular material further comprise a quantity of water from 1.0 to 50 mg per g of granules or granular material.

2. Granules or granular material according to claim 1, wherein the gum is acacia gum.

3. Granules or granular material according to one of the preceding claims, wherein the gum or gums are present at the minimum concentration of 1 mg per g of granules or granular material, preferentially at the minimum concentration of 10 mg per g of granules or granular material and more preferentially at the minimum concentration of 40 mg per g of granules or granular material.

4. Granules or granular material according to one of the preceding claims, wherein the effervescence-generating compound represents at least 50 mg per g of granules or granular material.

5. Granules or granular material according to one of the preceding claims comprising from 10 to 30 mg of water per g of granules or granular material.

6. Granules or granular material according to one of the preceding claims, further comprising one or several secondary compounds.

7. Granules or granular material according to claim 6, wherein the secondary compound is lactose.

8. Effervescent granules or granular material according to one of the preceding claims, able to be obtained according to a method of manufacturing comprising a step of spraying a non-aqueous organic solvent into which has been introduced the quantity of water retained.

9. Granules or granular material according to claim 8, **characterised in that** the method of manufacturing is implemented by means of a fluidised bed granulator, with the non-aqueous organic solvent into which has been introduced the quantity of water retained being sprayed onto the composition put into suspension in an air current by means of a nozzle.

10. Granules or granular material according to one of claims 1 to 7, able to be obtained by compaction by means of a compactor wherein the quantity of water is finely sprayed onto the mixture of the other constituents before compaction, with the resulting compacted material then being crushed and calibrated.

11. Granules or granular material according to claim 10 for its application as a drug.

12. Use of granules or granular material according to any of claims 1 to 10 in the cosmetics, or farming and horticulture, or hygiene, or maintenance field.

13. Use of granules or granular material according to one of claims 1 to 10, for the preparation of supplemented liquid feed for animals wherein said granules or granular material has been incorporated.

14. Use of granules or granular material according to one of claims 1 to 10, for the preparation of supplemented drinking water for animals wherein said granules or granular material has been incorporated.

15. Supplemented feed for animals incorporating granules or granular material according to one of claims 1 to 10.

16. Use of granules or granular material according to one of claims 1 to 10 for the dipping of aquatic organisms by dissolution of said granules or granular material in the aquatic environment.

17. Method for manufacturing granules or granular material according to one of claims 1 to 10 by granulation, comprising, in order, the steps of:
mixing powdery components of said granules or granular material, including gum, in order to obtain a mixture, spraying, onto said mixture, of an organic solvent into which has been introduced the quantity of water; and
removing said solvent via evaporation, **characterised in that** the mixture whereon is sprayed the water does not comprise an effervescence-generating compound or does not comprise any member of the pair of effervescence-generating compounds when it entails a pair.
